# EUROPEAN PATENT SPECIFICATION

(11) **EP 0 724 412 B1**
(45) Date of publication and mention of the grant of the patent: **26.05.1999**
(21) Application number: 94931856.2
(22) Date of filing: 12.10.1994
(51) Int. Cl.: A61B 19/08

(54) **SURGICAL DRAPE AND METHOD OF ASSEMBLY**
CHIRURGISCHES ABDECKTUCH UND VERFAHREN ZU DESSEN ANORDNUNG
CHAMP CHIRURGICAL ET SON PROCEDE D'ASSEMBLAGE

(30) Priority: 18.10.1993 US 138156
(43) Date of publication of application: 07.08.1996
(73) Proprietor: KIMBERLY-CLARK WORLDWIDE, INC., Neenah, Wisconsin 54956 (US)
(72) Inventor: CARLSON, Gerald, Ingersoll, II, Marietta, GA 30066 (US)
(74) Representative: DIEHL GLAESER HILTL & PARTNER
(86) International application number: US9411721
(87) International publication number: WO9510986

(56) References cited:
- EP-A- 0 140 857
- EP-A- 0 619 099
- WO-A-81/02100
- US-A- 3 503 391
- US-A- 3 799 161
- US-A- 4 024 862
- US-A- 5 109 873
- US-A- 5 222 507
- US-A- 5 345 946

## Description

### FIELD OF INVENTION

The present invention relates to surgical drapes and more particularly, to surgical drapes having surgical procedure attachments secured thereto.

### BACKGROUND OF THE INVENTION

As is generally known, sterile surgical drapes can be designed to greatly reduce, if not prevent, the transmission through the draping material of liquids and biological contaminates which may become entrained therein. In such surgical procedure environments, liquid and biological contaminates include, for example, operating room personnel perspiration, patient liquids, such as blood, and life support liquids, such as plasma and saline.

In the past, reusable surgical drapes were made of cotton or linen and were sterilized prior to use in the operating room. These materials however permitted transmission or "strike-through" of various liquids encountered in surgical procedures. In these instances, a path was established for transmission of bacteria and other contaminates to and from the patient.

Disposable surgical drapes, which are also sterilized prior to use in the operating room, have largely replaced the reusable surgical drapes, such as linen and cotton surgical drapes. Many disposable surgical drapes are customized to be compatible with specific procedures such as neurological, obstetric, orthopedic, cardiovascular and ophthalmic procedures. Several procedures, such as eye, abdominal, limb and back surgery may be performed on either the right- or the left-hand side of the body, depending upon the ailment or injury. As a result of the number of specific procedures requiring drape customizing, as well as the inherent dexterity requirements of each, many different variations of customized drapes must be stocked to accommodate these specific procedures. The alternative to customizing is a cumbersome single drape design used to cover all options Physician preference for preforming surgical procedures from the patients right side or left side also contributes to the quantity of stocked customized draping.

US-A-4,024,862, e.g. describes a drape comprising a flexible main sheet having a fenestration and a flexible frame sheet removably secured to the upper surface of the drape around the fenestration of a size smaller than and aligned with the drape fenestration.

Additionally, such customization may further include incorporating various surgical procedure attachments or "work stations" into the drape about the operating site. Such surgical procedure attachments include, tubes, hoses, fluid collecting and channeling structures, tube holders, non-skid surfaces, laser shielding, absorbent surfaces, fabric reinforcement, attachment tape, clipping tabs, retractor tabs, and adhesive incise. The addition of such attachments or work stations to the surgical drape further taxes storage space, costs, material usage and disposal concerns. Therefore, there exists a need for a surgical drape or a surgical drape system which provides the "customization" required by the surgical community, is storage friendly, and is responsive to efficient resource management, such as material resources, acquisition resources and disposal resources.

### SUMMARY OF THE INVENTION

The inventor, conscious of the above mentioned problems with conventional surgical drapes, has studied the life cycle of conventional surgical drapes and draping systems from manufacture to disposal. Particularly, the inventor has studied the manufacturing process of conventional drapes and has noted the large assembling stations required to customize conventional drapes, particularly about the operating site, in addition to the time assemblers exhausted simply handling and having to work around peripheral drape material.

The inventor has further observed and appreciated that during most surgical procedures, the portion or area of the drape dedicated to the operating site or area of penetration generally is of an area less than about one half and in some cases substantially less than about one half of the total area of the drape. Furthermore, with improved fluid containment systems and other such surgical procedure attachments previously mentioned secured to the drape about the area of operation, the inventor has observed that such attachments significantly reduce or prevent the incident of insult or contact of liquids to the area or portion of the gown outboard of the operating site.

The inventor has further observed that during surgery there occurs from time to time a need to replace a surgical procedure attachment or re-configure surgical procedure attachments. These observations and other observations by the inventor, such as drape storage, disposal and acquisition have led the inventor to conceive the present invention.

In response to the above difficulties and problems encountered with conventional drapes a new surgical drape has been fashioned. More particularly, the present invention relates to a modular surgical drape having a first drape portion and a second drape portion. A means is provided for selective coupling and uncoupling the second drape portion to the first drape portion.

The first drape portion is a continuous sheet of material and is provided with separable sections. The separable sections may be located about the first drape portion so that the dimension of or location of an opening therein may be affected upon the separation of one or more separable sections. The first drape portion may also include portions thereof which are reinforced by a distinct layer of material.

The first drape portion and second drape portion may be formed from similar material, such as either all disposable material or all reusable material. Alternatively, the first drape portion and the second drape portion may be made of reusable and disposable material. For example, the first drape portion may be made of a reusable or re-sposable (limited reuse) material and the second drape portion made of disposable material. Additionally, the first drape portion may be made of a disposable material and the second drape portion may be made of reusable material. The present invention may further include work stations and/or surgical procedure attachments. These work stations and surgical procedure attachments may be positioned on the second drape portion or on a work station panel which is positioned on the second drape portion.

### BRIEF DESCRIPTION OF THE DRAWINGS

FIG. 1 is a front plan view of an embodiment of a first drape portion illustrating separable sections.
FIG. 2 is a front plan view of another embodiment of the present invention illustrating a first drape portion with separable sections and a second drape portion.

### DETAILED DESCRIPTION OF THE INVENTION

Several terms are used herein to refer to various parts of the surgical drape as the drape is positioned about the operating room and patient. The "front" refers to that part of the drape which faces away from the patient. The "back" refers to that part of the drape which faces the patient. The "side" or "sides" refer to that part of the drape which overlay the side or lateral portions of the patient.

Additionally, several terms are used herein to refer to attaching, detaching and reattaching one or more parts of the drape to another part of the drape such that these parts may be selectively coupled, uncoupled and recoupled to one another. These terms include "detachably joined", "detachably secured", "detachably attached" and derivatives and synonyms thereof. Such coupling of these drape parts to one another may be accomplished by several conventional methods. By way of example and not limitation, these methods include zipping, snapping, taping, using matingly engaging hook and loop fasteners, such as VELCRO®, buttons, magnetic fasteners, cohesive adhesives, adhesives and other such detachable coupling and reattachable coupling methods familiar to those skilled in the art.

It is noted that either the first drape portion or second drape portion may be made from a multitude of disposable and/or re-sposable materials including nonwoven materials. In some embodiments, a stretchable nonwoven material may be used. A material well-suited for use with the present invention is a three-layer nonwoven polypropylene material known as SMS. SMS is an acronym for spunbond, meltblown, spunbond. See for example, U.S. Patent No. 4,041,203 to Brock et al. One particular advantage is that the SMS material exhibits enhanced fluid barrier characteristics. It should be noted, however, that other disposable nonwovens as well as other materials including wovens, films, foam/film laminates and combinations thereof may be used in the present invention. It is also contemplated that either or both the first drape portion and the second drape portion may be coated with a liquid impervious coating to prevent fluid absorption or, alternatively, the second drape portion may be made of one or more absorbent material layers.

As previously mentioned, the drape may be made from a combination of disposable materials and reusable materials or formed totally from reusable materials or totally from disposable materials. Examples of suitable reusable materials include, but are not limited to, cotton, linen, polyester, woven polyester and polytetrafluoroethylene.

In a dimensionalized drape the outer edges of the first drape portion are similar to a conventional laparoscopy drape. A second drape portion is assumed to abut the edges of the first drape portion defining the opening. A conventional drape formed from SMS polypropylene having a fabric weight of 1.59 oz/sq.yd. and sized to dimensions indicated for the outer edges of the first drape portion would have a total drape area of 9,654 square inches and an approximate weight of around 16 ounces. Such a conventional drape, after contact by biomedical waste, would require the entire 9,654 square inches of material to be treated as biomedical waste.

By comparison, while the drape of the present invention formed from the same material would also have a total area of about 9,654 square inches and weigh around 16 ounces, the area of the first drape portion is around 7,548 square inches and weighs about 11 ounces and the area of the second drape portion is around 2,106 square inches and weighs about 5 ounces. By confining the biomedical waste to the second drape portion, the volume and weight of drape material to be treated via biomedical waste procedures is reduced by around 70% over conventional drapes. Similar calculations may be performed to illustrate enhanced storage and material savings over conventional drapes.

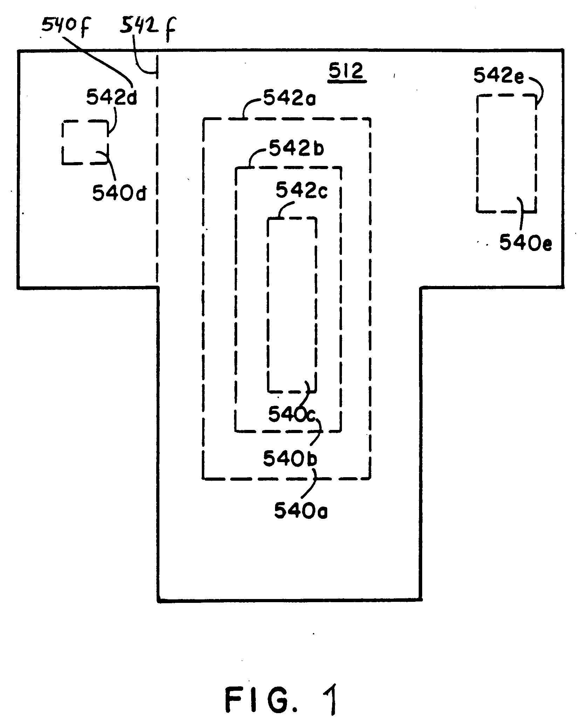

Turning now to FIG. 1, a first drape portion 512 is illustrated. The first drape portion 512 is a continuous sheet of material and includes a plurality of separable sections or tear-away sections 540 a-f. The term "continuous sheet" describes a layer of material which is formed from a single piece of material or formed by affixing two or more pieces of material, such as by sewing or gluing.

Borders of each separable section, 542 a-f, respectively, may be thinned, perforated or die-cut so as weaken the first drape portion 512 material along said borders. In this way, one may select the size and location of an opening corresponding to an operating site on the first drape portion 512 and remove the appropriate separable section by grasping and applying sufficient urging force so as to separate the selected separable section from the first drape portion 512. Once the location and size is determined and the appropriate separable section removed, the second drape portion (not shown in FIG. 1) may be secured to the first drape portion 512 as previously described.

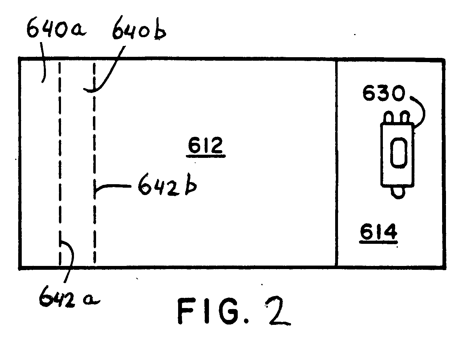

Referring now to FIG. 2, a first drape portion 612 with a second drape portion 614 attached thereto Is illustrated. A workstation 630, may be detachably secured to the second drape portion 614.

The work station 630 overlies the second drape portion 614. Generally, the work station 630 may include one or more surgical procedure attachments. The work station 630 or surgical procedure attachment(s) may be affixed to the second drape portion 614 by permanent means or the workstation 630 may be detachably secured by the means previously described for detachably securing the first and second portions.

The work station 630 may include a work station panel having an opening or opening cutout patterns. The work station panel may be detachably secured to the second drape portion 614 such that the opening of the workstation panel vertically aligns with an opening or an opening cutout pattern in the second drape portion 614. Several surgical procedure attachments, as previously discussed, may be affixed to the work station panel about the opening. It will be further appreciated, though not illustrated, that in some applications, such surgical procedure attachments may be affixed to the second drape portion 614. In this case, the need for the work station panel may be avoided.

As previously mentioned, the surgical procedure attachments, such as those mentioned above, may be included on the second drape portion 614. In this way, the second drape portion 614 may be configured for a selected procedure and coupled to the first drape portion 612. As surgical procedure attachment requirements change during the operation, the second drape portion 614 may be uncoupled and a properly configured second drape portion 614 coupled to the first drape portion 612. Alternatively, individual surgical procedure attachments may be removed from either the second drape portion 614 or work station panel during the surgical procedure as surgical procedure attachment requirements change. In this way, during the process of reconfiguring or changing surgical procedure attachments, only a fraction of thee surgical draping material is disturbed during surgery.

The first drape portion 612 is a continuous sheet of material and includes a plurality of separable sections or tear-away sections 640 a and b. The borders of each separable section, 642 a and b respectively, may be thinned, perforated or die-cut so as to weaken the material of the first drape portion 612 along said borders. In this way, the length or width of the first drape portion 612 may be quickly and selectively altered by separating the appropriate separable section from the first drape portion 612 as described above.

Several advantages of the present invention are readily apparent. For example, as the drape of the present invention is modular, the first drape portion can be of a uniform or a "one-size-fits-all" variety. As such, customization is predominately confined to the second drape portion, which is generally sized to overlie the operating site. Modularity reduces manufacturing cost and time. This is so because generally, the second drape portion alone requires customizing and the securing thereon of surgical procedure attachments. The second drape portion being generally of smaller dimension than that of the first drape portion or conventional drapes allows the second drape portion to be assembled on smaller work surfaces and in absence of the otherwise cumbersome and bulky non-operating site drape material.

Modularity also permits the drape purchaser to more efficiently allocate drape acquisition budgets by purchasing fewer first drape portions than second drape portions. This is so because, the first drape portion or non-operating site material can accommodate a plurality of different second drape portions. For example, rather than purchasing, storing and maintaining one left- and one right-handed conventional drape, one first drape portion and two second drape portions, one left- and one right-handed, are required. Additionally, the first drape portion may be of such dimension such that a single first drape portion may be suitable for combination with several second drape portions. By using an appropriate mix of first drape portions and second drape portions provided by the present invention such a purchaser may still maintain the flexibility to meet a host of surgical demands, which otherwise, in the case of conventional drapes, would require acquisition and storage of a one to one ratio of non-operating site material with operating site material. First drape portions with separable sections further improve such supply and demand flexibility.

Modularity further provides the purchaser with a reusable option. For example, a reusable option might include a first drape portion constructed of a reusable material able to withstand re-sterilization procedures with the second drape portion formed from a disposable material. As previously mentioned, work stations, and particularly fluid control systems, confine fluid contact with the drape to the operating site. In this way, re-sterilization and reuse of the first drape portion may be more attractive.

Additionally, whether the first drape portion and second drape portion are formed from disposable materials or, as above, the first drape portion is formed from a reusable material, the present invention provides improved biomedical waste management over convention systems. This is so because the drape of the present invention can be design to make it highly likely that only the second drape portion is insulted. As such, the insulted and non-insulted portions of the drape can be separated. Separability allows the operating site portion of the drape to be disposed or processed via a biomedical waste treatment route while the non-insulted non-operating site material may be disposed or processed via a non-biomedical waste treatment route.

Furthermore, modularity of the present invention offers the health care provider or intermediary drape supplier with the option of assembling or configuring the drape on-site or off-site. As the second drape portion, which is generally smaller in size and easer to handle, receives the majority of the customizing effort, on-site or off-site customizing or on-site or off-site re-configuring is now more attractive.

While the invention has been described in detail with respect to specific embodiments thereof, it will be appreciated that those skilled in the art, upon attaining an understanding of the foregoing, may readily conceive of alterations to, variations or and equivalents to these embodiments. Accordingly, the scope of the present invention should be assessed as that of the appended claims and any equivalents thereto.

## Claims

1. A modular surgical drape comprising:
a first drape portion (512;612);
a second drape portion (614); means for selectively coupling and uncoupling said second drape portion (614) to said first drape portion (512;612)
characterized in that
said first drape portion (512;612) is a continuous sheet of material including a plurality of separable sections (540a, 540b,540c,540d,540e,540f,640a,640b).

2. The surgical drape of claim 1 wherein said first drape portion (512) comprises means (542a,542b,542c,542d,542e,642a,642b) for separating said separable sections (540a,540b,540c,540d,540e,540f,640a,640b) from said first drape portion (512,612).

3. The modular surgical drape of claim 1 or 2 wherein the first drape portion (512;612) is formed from a reusable material and the second drape portion (614) is formed from a disposable material.

4. The modular surgical drape of one of claims 1 to 3 wherein the first drape portion (512;612) further includes a layer of reinforcing material.

5. The modular surgical drape of one of claims 1 to 4 wherein the first drape portion (612) includes a front surface and the second drape portion (614) includes peripheral edges, and wherein portions of the second drape portion (614) adjacent the peripheral edges overly portions of the front surface of the first drape portion (612) around the opening when the second drape portion (614) is selectively coupled to the first drape portion (612).

6. The modular surgical drape of one of claims 1 to 5 wherein the second drape portion (614) further includes a fenestration therein.

7. The modular surgical drape of one of claims 1 to 6 wherein said first drape portion (512;612) has a front surface, a back surface, and peripheral edges;
said second drape portion (614) has a front surface, a back surface and peripheral edges;
wherein said second drape portion (614) is adapted for said selective coupling and uncoupling to the first drape portion (512;612); and
wherein the second drape portion (614) is adapted to receive surgical procedure attachments (630).

8. The modular surgical drape of claim 7 wherein portions of the second drape portion (614) adjacent the peripheral edges overly portions of the front surface of the first drape portion (512;612) around the opening defined by the first drape portion (512;612) when the second drape portion (614) is coupled to the first drape portion (512;612).

9. A method of assembling a drape comprising:
providing a first drape portion and a second drape portion, wherein the first drape portion and the second drape portion are adapted for selective coupling and uncoupling and wherein said first drape portion is a continuous sheet of material;
removing a separable section from said first drape portion; and
coupling the second drape portion to the first drape portion.

10. The method of claim 9 wherein the first drape portion defines an opening and wherein the portions of the first drape portion about the opening are adapted for selective coupling and uncoupling of the second drape portion thereto.

## Patentansprüche

1. Modularer chirurgischer Behang mit:
einem ersten Behangteil (512; 612);
einem zweiten Behangteil (614);
Mitteln, um selektiv den zweiten Behangteil (614) mit dem ersten Behangteil (512; 612) zu verbinden und hiervon zu trennen,
dadurch gekennzeichnet, daß
der erste Behangteil (512; 612) eine kontinuierliche Schicht eines Materials mit einer Mehrzahl trennbarer Abschnitte (540a, 540b, 540c, 540d, 540e, 540f, 640a, 640b) ist.

2. Chirurgischer Behang gemäß Anspruch 1, bei dem der erste Behangteil (512) Mittel (542a, 542b, 542c, 542d, 542e, 642a, 642b) zum Trennen der trennbaren Schichten (540a, 540b, 540c, 540d, 540e, 540f, 640a, 640b) von dem ersten Behangteil (512; 612) aufweist.

3. Modularer chirurgischer Behang gemäß Anspruch 1 oder 2, bei dem der erste Behangteil (512; 612) aus einem wiederverwendbaren Material gebildet ist und der zweite Behangteil (614) aus einem Wegwerfmaterial gebildet ist.

4. Modularer chirurgischer Behang gemäß einem der Ansprüche 1 bis 3, bei dem der erste Behangteil (512; 612) des weiteren eine Schicht aus Verstärkungsmaterial enthält.

5. Modularer chirurgischer Behang gemäß einem der Ansprüche 1 bis 4, bei dem der erste Behangteil (612) eine Vorderfläche und der zweite Behangteil (614) periphere Kanten aufweist, und wobei Bereiche des zweiten Behangteils (614) benachbart zu den peripheren Kanten über Bereichen der Vorderfläche des ersten Behangteils (612) um die Öffnung herum liegen, wenn der zweite Behangteil (614) selektiv mit dem ersten Behangteil (612) verbunden ist.

6. Modularer chirurgischer Behang gemäß einem der Ansprüche 1 bis 5, bei dem der zweite Behangteil (614) hierin des weiteren eine Befensterung aufweist.

7. Modularer chirurgischer Behang gemäß einem der Ansprüche 1 bis 6, bei dem der erste Behangteil (512; 612) eine Vorderfläche, eine Rückfläche und periphere Kanten aufweist;
der zweite Behangteil (614) eine Vorderfläche, eine Rückfläche und periphere Kanten aufweist;
wobei der zweite Behangteil (614) für die selektive Verbindung mit und Trennung von dem ersten Behangteil (512; 612) geeignet ist; und
wobei der zweite Behangteil (614) zur Aufnahme von Chirurgieverfahrensanbringungen (630) geeignet ist.

8. Modularer chirurgischer Behang gemäß Anspruch 7, bei dem Bereiche des zweiten Behangteils (614) benachbart zu den peripheren Kanten über Bereichen der Vorderfläche des ersten Behangteils (512; 612) um die Öffnung herum, definiert durch den ersten Behangteil (512; 612), liegen, wenn der zweite Behangteil (614) mit dem ersten Behangteil (512; 612) verbunden ist.

9. Verfahren zur Zusammensetzung eines Behangs, das folgendes umfaßt:
Bereitstellung eines ersten Behangteils und eines zweiten Behangteils, wobei der erste Behangteil und der zweite Behangteil zur selektiven Verbindung und Trennung geeignet sind, und wobei der erste Behangteil eine kontinuierliche Materialschicht ist;
Entfernung eines trennbaren Abschnitts von dem ersten Behangteil; und
Verbindung des zweiten Behangteils mit dem ersten Behangteil.

10. Verfahren gemäß Anspruch 9, bei dem der erste Behangteil eine Öffnung definiert, und wobei die Bereiche des ersten Behangteils um die Öffnung herum zur selektiven Verbindung des zweiten Behangteils hiermit und Trennung des zweiten Behangteils hiervon geeignet sind.

## Revendications

1. Champ chirurgical modulaire comprenant :
une première portion de champ chirurgical (512;612) ;
une seconde portion de champ chirurgical (614) ;
un moyen pour connecter/déconnecter sélectivement ladite seconde portion de champ chirurgical (614) à ladite première portion de champ chirurgical (512;612)
caractérisé en ce que
ladite première portion de champ chirurgical (512;612) est une feuille continue de matériau comprenant une série de sections séparables (540a,540b,540c, 540d,540e,540f,640a,640b).

2. Champ chirurgical selon la revendication 1, dans lequel ladite première portion de champ chirurgical (512) comprend des moyens (542a,542b,542c, 542d,542e,642a,642b) pour séparer lesdites sections séparables (540a,540b,540c,540d,540e,540f,640a,640b) de ladite première portion de champ chirurgical (512;612).

3. Champ chirurgical modulaire selon la revendication 1 ou 2, dans lequel la première portion de champ chirurgical (512;612) est formée à partir d'un matériau réutilisable et la seconde portion de champ chirurgical (614) est formée à partir d'un matériau jetable.

4. Champ chirurgical modulaire selon l'une des revendications 1 à 3, dans lequel la première portion de champ chirurgical (512;612) comprend en outre une couche d'un matériau de renforcement.

5. Champ chirurgical modulaire selon l'une des revendications 1 à 4, dans lequel la première portion de champ chirurgical (612) comprend une face avant et la seconde portion de champ chirurgical (614) comprend des bords périphériques, et dans lequel des portions de la seconde portion de champ chirurgical (614) adjacentes aux bords périphériques chevauchent des portions de la face avant de la première portion de champ chirurgical (612) autour de l'ouverture lorsque la seconde portion de champ chirurgical (614) est sélectivement connectée à la première portion de champ chirurgical (612).

6. Champ chirurgical modulaire selon l'une des revendications 1 à 5, dans lequel la seconde portion de champ chirurgical (614) comprend en outre une fenêtre ménagée dans celle-ci.

7. Champ chirurgical modulaire selon l'une des revendications 1 à 6, dans lequel ladite première portion de champ chirurgical (512;612) présente une face avant, une face arrière et des bords périphériques ; et
ladite seconde portion de champ chirurgical (614) présente une face avant, une face arrière et des bords périphériques ;
ladite seconde portion de champ chirurgical (614) étant adaptée à ladite connexion/déconnexion sélective à ladite première portion de champ chirurgical (512;612) ; et
la seconde portion de champ chirurgical (614) étant adaptée à recevoir des accessoires pour procédures chirurgicales (630).

8. Champ chirurgical modulaire selon la revendication 7, dans lequel des portions de ladite seconde portion de champ chirurgical (614) adjacentes aux bords périphériques chevauchent des portions de la face avant de la première portion de champ chirurgical (512;612) autour de l'ouverture définie par la première portion de champ chirurgical (512;612) lorsque la seconde portion de champ chirurgical (614) est connectée à ladite première portion de champ chirurgical (512;612).

9. Procédé d'assemblage d'un champ opératoire comprenant :
la fourniture d'une première portion de champ chirurgical et d'une seconde portion de champ chirurgical, la première portion de champ chirurgical et la seconde portion de champ chirurgical étant adaptées à une connexion/déconnexion sélective et ladite première portion de champ chirurgical étant une feuille continue de matériau ;
l'enlèvement d'une section séparable de ladite première portion de champ chirurgical ; et
la connexion de la seconde portion de champ chirurgical à la première portion de champ chirurgical.

10. Procédé selon la revendication 9, dans lequel la première portion de champ chirurgical définit une ouverture et dans lequel les portions de la première portion de champ chirurgical situées autour de l'ouverture sont adaptées à la connexion/déconnexion sélective de la seconde portion de champ chirurgical auxdites portions.
